(19) Europäisches Patentamt
European Patent Office
Office européen des brevets

(11) Veröffentlichungsnummer: **0 137 309**
**B1**

# (12) EUROPÄISCHE PATENTSCHRIFT

(45) Veröffentlichungstag der Patentschrift: **29.06.88**

(51) Int. Cl.⁴: **C 07 D 319/06**, C 08 G 63/62

(21) Anmeldenummer: **84110591.9**

(22) Anmeldetag: **06.09.84**

(54) **Mehrcyclische Kohlensäureester und neue Copolymere erhältlich aus diesen mehrcyclischen Kohlensäureestern und monocyclischen Carbonaten.**

(30) Priorität: **16.09.83 DE 3333413**

(43) Veröffentlichungstag der Anmeldung:
**17.04.85 Patentblatt 85/16**

(45) Bekanntmachung des Hinweises auf die Patenterteilung:
**29.06.88 Patentblatt 88/26**

(84) Benannte Vertragsstaaten:
**DE FR GB IT**

(56) Entgegenhaltungen:
**EP-A-0 057 360**

(73) Patentinhaber: **BAYER AG, Konzernverwaltung RP Patentabteilung, D-5090 Leverkusen 1 Bayerwerk (DE)**

(72) Erfinder: **Mues, Peter, Dr., Breslauer Strasse 31, D-4150 Krefeld 11 (DE)**
Erfinder: **Brassat, Bert, Dr., Bethelstrasse 24, D-4150 Krefeld (DE)**
Erfinder: **Buysch, Hans- Josef, Dr., Brandenburger Strasse 28, D-4150 Krefeld (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 137 309 B1

LIBER, STOCKHOLM 1988

**Beschreibung**

Aus der EP-A2 0 057 360 sind Kohlensäurederivate bekannt, die der Formel (VII) entsprechen

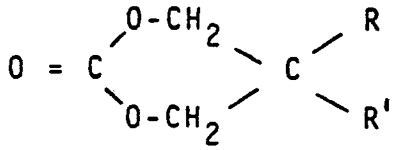 (VII).

In Formel (VII) bedeutet R
entweder $-CH_2OH$, wobei dann R' für einen Alkylrest mit 1 bis 4 C-Atomen steht,
oder

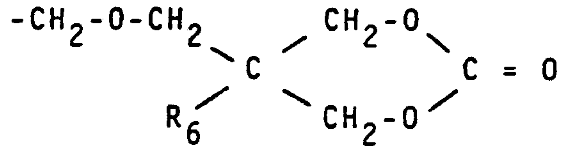,

wobei R' und $R_6$ unabhängig voneinander für Methyl oder Ethyl stehen,
oder

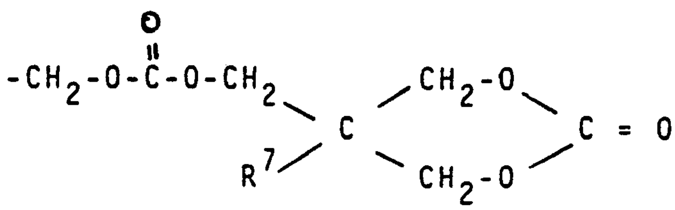,

wobei R' und $R_7$ jeweils für einen Alkylrest mit 1 bis 4 C-Atomen stehen,
oder
in der R und R' gemeinsam

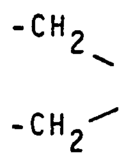

bedeuten, wobei A für die Gruppe

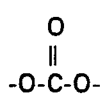

oder für Sauerstoff steht. Aus der EP-A2 0 057 360 ist ferner ein Verfahren zur Herstellung von Kohlensäurederivaten der Formel (VII) bekannt und die Verwendung derartiger Kohlensäurederivate zur Herstellung von Polycarbonaten.

Die vorliegende Erfindung betrifft mehrcyclische Kohlensäureester der Formel (III)

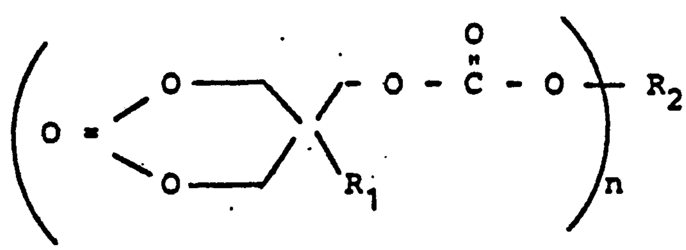 (III),

in der

$R_1$ für einen Alkylrest mit 1 bis 4 C-Atomen steht,

$R_2$ für einen aliphatischen Kohlenwasserstoffrest mit 3 bis 18 C-Atomen, für einen aromatischen Kohlenwasserstoff-Rest mit 6 bis 12 C-Atomen oder für einen araliphatischen Kohlenwasserstoff-Rest mit 7 bis 24 C-Atomen steht und

n 2, 3 oder 4 bedeutet.

In der Formel (III) steht $R_1$ vorzugsweise für Methyl oder Ethyl und $R_2$ vorzugsweise für das Kohlenwasserstoffgerüst eines der folgenden 2- bis 4-wertigen Alkohole: Trimethylolethan, Trimethylolpropan, Pentaerythrit, Butandiol-1,4, Buten-2-diol-1,4, Butin-2-diol-1,4, Neopentylglykol, Hexandiol-1,6, 2-Methylen-propandiol-1,3, Cyclohexandiol-1,4, Cyclohexan-1,4-dimethanol, Bisphenol A und Perhydrobisphenol A. n entspricht dann der Wertigkeit des Alkohols, dessen Kohlenwasserstoffgerüst den Rest $R_2$ darstellt. Besonders bevorzugt ist $R_2$ das Kohlenwasserstoffgerüst eines 2- oder 3-wertigen Alkohols, ganz besonders bevorzugt das Kohlenwasserstoffgerüst eines zweiwertigen Alkohols, d. h. n steht besonders bevorzugt für 2 oder 3 und ganz besonders bevorzugt für 2.

Die mehrcyclischen Kohlensäureester der Formel (III) können beispielsweise hergestellt werden, indem man Chlorkohlensäureester der Formel (I)

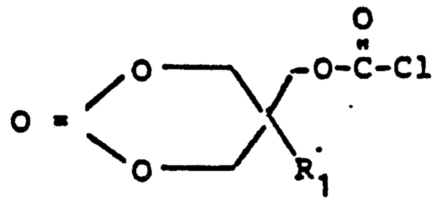

(I),

in der

$R_1$ für einen Alkylrest mit 1 bis 4 C-Atomen steht,

zu einer Lösung, die einen 2- bis 4-wertigen Alkohol und ein säurebindendes Mittel enthält, nach und nach bei Temperaturen im Bereich von 0 bis 30°C zufügt.

Vorzugsweise steht in Formel (I) $R_1$ für eine Methyl- oder Ethylgruppe.

Die Herstellung der Chlorkohlensäureester der Formel (I) kann durch Phosgenierung der den Chlorkohlensäureestern zugrunde liegenden Alkohole der Formel (II)

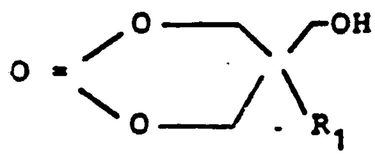

(II),

in der

$R_1$ die oben angegebene Bedeutung hat,

nach einem an sich bekannten Verfahren erfolgen. Die Alkohole der Formel (II) können gemäß dem in der DE-OS-3 103 139 beschriebenen Verfahren hergestellt werden.

Die Phosgenierung der Alkohole der Formel (II) kann beispielsweise so erfolgen, daß man Phosgen in einem Lösungsmittel vorlegt und den Alkohol der Formel (II) zusammen mit einem säurebindenden Mittel nach und nach bei Temperaturen im Bereich von -5 bis +5°C zufügt. Phosgen wird dabei vorzugsweise im Überschuß eingesetzt, beispielsweise in 5 bis 20 %-igem molarem Überschuß. Lösungsmittel können beispielsweise chlorierte Kohlenwasserstoffe sein, wie Methylenchlorid. Als säurebindende Mittel kommen beispielsweise aromatische oder aliphatische Amine in Frage, wie N,N-Dimethylanilin, Pyridin oder Triethylamin. Beispiele für einzusetzende Alkohole der Formel (II) sind Trimethylolethancarbonat, Trimethylolpropancarbonat, Trimethylolbutancarbonat und Trimethylolpentancarbonat.

Die Isolierung der Chlorkohlensäureester der Formel (I) aus dem Phosgenierungs-Reaktionsgemisch kann beispielsweise dadurch erfolgen, daß man aus dem Reaktionzgemisch alle leicht flüchtigen Anteile durch Vakuumdestillation entfernt, den Rückstand in einem Lösungsmittel für die Chlorkohlensäureester der Formel (I), z. B. Toluol, aufnimmt, unlösliche Bestandteile abtrennt und das Lösungsmittel wieder entfernt.

Die Chlorkohlensäureester der Formel (I) sind Gegenstand einer Teilanmeldung.

Zur Herstellung der neuen mehrcyclischen Kohlensäureester der Formel (III) ist es nicht unbedingt erforderlich, die Chlorkohlensäureester der Formel (I) in reiner Form einzusetzen; sie können beispielsweise auch in Form der bei ihrer Herstellung anfallenden Roh-Lösung eingesetzt werden. Die 2- bis 4-wertigen Alkohole sind solche, deren Kohlenwasserstoffgerüst einen aliphatischen Kohlenwasserstoff-Rest mit 3 bis 18 C-Atomen, einen aromatischen Kohlenwasserstoff-Rest mit 6 bis 12 C-Atomen oder einen araliphatischen Kohlenwasserstoff-Rest mit 7 bis 24 C-Atomen darstellt. Vorzugsweise handelt es sich bei den 2- bis 4-wertigen Alkoholen um einen Alkohol aus der Gruppe Trimethylolethan, Trimethylolpropan, Pentaerythrit, Butandiol-1,4, Buten-2-diol-1,4, Butin-2-diol-1,4, Neopentylglykol, Hexandiol-1,6, 2-Methylen-propandiol-1,3, Cyclohexandiol-1,4, Cyclohexan-1,4-dimethanol, Bisphenol A und Perhydrobisphenol A. Als säurebindende Mittel kommen beispielsweise aromatische oder aliphatische Amine in Frage, wie N,N-Dimethylanilin, Pyridin oder Triethylamin. Lösungsmittel können beispielsweise chlorierte Kohlenwasserstoffe sein, wie Methylenchlorid.

Die Isolierung und Reinigung der mehrcyclischen Kohlensäureester der Formel (III) kann beispielsweise erfolgen, indem man aus dem Reaktionsgemisch zunächst das aus dem säurebindenden Mittel entstandene Hydrochlorid mit Wasser oder einer wäßrigen Salzlösung extrahiert, aus der verbleibenden organischen Phase,

gegebenenfalls nach einer Trocknung, das Lösungsmittel abtrennt und das so erhaltene Rohprodukt aus einem geeigneten Lösungsmittel, z. B. Butylacetat, kristallisiert.

Die vorliegende Erfindung betrifft weiterhin Copolymere, die durch Copolymerisation der mehrcyclischen Kohlensäureester der Formel (III) mit monocyclischen Carbonaten der Formel (IV)

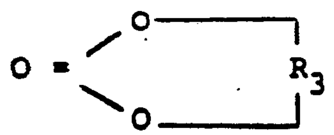 (IV) ,

in der

$R_3$ für $(-CH_2-)_m$, wobei m 3, 4, 5 oder 6 bedeutet, $-CH_2-CH=CH-CH_2-$, $-CH_2-CH_2-CH(CH_3)-$, $-CH_2-CH_2-O-CH_2-$, $CH_2-O-CH_2-CH_2-$,

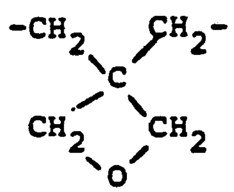 , 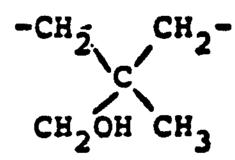 , 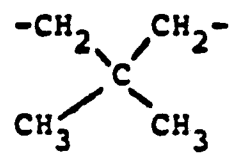

oder

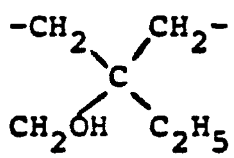

steht, oder der Formel (V)

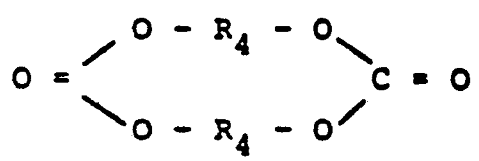 (V),

in der

$R_4$ für $(-CH_2-)_p$, wobei p eine ganze Zahl von 4 bis 12 bedeutet, $-CH_2-CH_2-O-CH_2-CH_2-$, $-CH_2-CH_2-O-CH_2-CH_2-O-CH_2-CH_2-$ oder
$-CH_2-CH_2-N-CH_2-CH_2-$ steht,
|
$CH_3$

oder der Formel (VI)

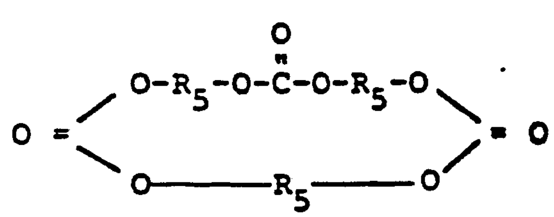 (VI),

in der

$R_5$ für $-CH_2-CH_2-O-CH_2-CH_2$ steht

und Einsetzen von 2 bis 50, vorzugsweise 5 bis 20 Gew.-% des mehrcyclischen Kohlensäureesters der Formel (III) und 98 bis 50, vorzugsweise 95 bis 80 Gew.-% des monocyclischen Carbonats (diese Gewichtsprozente beziehen sich jeweils auf die Gesamtmenge eingesetzter organischer Carbonate), erhältlich sind.

Die Herstellung monocyclischer Carbonate der Formeln (IV), (V) und (VI) ist beispielsweise in den DE-OS'en 31 03 135, 31 03 139, 31 03 140 und 32 04 078 beschrieben.

Vorzugsweise werden monocyclische Carbonate der Formel (IV) eingesetzt, besonders bevorzugt 5,5-Dimethyl-1,3-dioxan-2-on.

Bei der Copolymerisation der mehrcyclischen Kohlensäureester der Formel (III) mit den monocyclischen Carbonaten wird der Ring der monocyclischen Carbonate geöffnet, und es entstehen lineare Polycarbonate, die durch von den neuen mehrcyclischen Kohlensäureestern der Formel (III) stammende Struktureinheiten vernetzt sind. Diese Copolymere sind Duromere mit verbesserten Eigenschaften, insbesondere mit überraschend hohen Schlagzähigkeiten.

Die Copolymerisation wird im allgemeinen bei erhöhter Temperatur durchgeführt, beispielsweise bei Temperaturen im Bereich von 80 bis 200°C. Bevorzugt sind Temperaturen im Bereich von 100 bis 160°C. Besonders bevorzugt sind Temperaturen im Bereich von 110 bis 140°C. Die Copolymerisation wird ferner im allgemeinen unter Zusatz von Katalysatoren durchgeführt. Geeignete Katalysatoren sind beispielsweise die Carbonate und Carboxylate der Metalle Lithium, Natrium, Kalium, Zinn, Blei und Thallium. Bevorzugt sind Carboxylate von Lithium, Blei und Thallium. Die Katalysatoren können beispielsweise in Mengen von 0,01 bis 0,1 Gew.-%, bezogen auf die Gesamtmenge eingesetzter Carbonate, eingesetzt werden. Bevorzugt sind Katalysatormengen von 0,01 bis 0,05 Gew.-%.

Die durch diese Copolymerisation zugänglichen Duromere können mit Vorteil überall dort eingesetzt werden, wo hohe Anforderungen an duromertypische Eigenschaften, wie Wärmeformbeständigkeit und Unempfindlichkeit gegen Agentien, die eine Lösung oder Quellung hervorrufen können (z. B. Kraft- und Schmierstoffe), gestellt werden und, wo zusätzlich gute mechanische Eigenschaften, insbesondere eine hohe Zähigkeit notwendig sind. Beispielsweise können Kraftfahrzeugteile aus derartigen Duromeren hergestellt werden, insbesondere solche, die der Gefahr von Steinschlag ausgesetzt sind, wie Spoiler, Stoßstangen(teile) und Kotflügel(auskleidungen).

Die folgenden Beispiele erläutern die Erfindung, ohne sie in irgendeiner Weise zu beschränken.

**Beispiele**

**Beispiel 1**

Zu einer Lösung von 53 g (0,535 Mol) Phosgen in 200 ml getrocknetem Methylenchlorid wurde bei 5°C innerhalb von 40 Min. eine Lösung von 80 g (0,5 Mol) 1,3-Dioxan-5-hydroxymethyl-5-ethyl-2-on und 39,5 g (0,5 Mol) Pyridin in 200 ml getrocknetem Methylenchlorid zugetropft. Die Mischung wurde bei 0°C während weiterer 3 Stunden gerührt. Anschließend wurden im Wasserstrahlvakuum 100 ml Methylenchlorid abdestilliert. Das zum Teil ausfallende Pyridinhydrochlorid wurde abgesaugt und mit wenig Methylenchlorid gewaschen. Das Filtrat wurde mittels eines Rotationsverdampfers bei 0°C eingeengt. Der kristalline Rückstand (163 g) wurde 3-mal mit insgesamt 1,6 l Toluol verrührt und die ungelösten Bestandteile abgesaugt. Das Filtrat wurde mittels eines Rotationsverdampfers bei 30°C eingeengt. Es verblieben 79 g (71 % der Theorie) kristallines Produkt mit einem Schmelzpunkt von 62 bis 65°C.

Das $^1H$-NMR-Spektrum und die analytische Chlorbestimmung bestätigten die Struktur und Zusammensetzung des erhaltenen Produkts als eine Verbindung der Formel (I) mit $R_1 = C_2H_5$.

**Beispiel 2**

Zu einer Lösung von 110 g (1,1 Mol) Phosgen in 400 ml getrocknetem Methylenchlorid wurden 160 g (1 Mol) 1,3-Dioxan-5-hydroxymethyl-5-ethyl-2-on gelöst in 95 g (1,2 Mol) Pyridin und 700 ml getrocknetem Methylenchlorid innerhalb von 2 Stunden bei 4°C zugetropft. Nach weiteren 2 Stunden wurde die erhaltene Lösung zu einer Mischung aus 82 g (0,75 Mol) Cyclohexan-1,4-dimethanol, 79 g (1 Mol) Pyridin und 200 ml Methylenchlorid bei 20°C innerhalb von 25 Min. gegeben. Nach 15 Stunden bei Raumtemperatur wurde 3-mal mit insgesamt 1 l gesättigter wäßriger NaCl-Lösung extrahiert. Die organische Phase wurde über $Na_2SO_4$ getrocknet und mittels eines Rotationsverdampfers schonend eingeengt (Badtemperatur 40°C). Restliches Lösungsmittel wurde im Vakuum einer Ölpumpe entfernt. Das Rohprodukt (286 g) wurde in 900 ml heißem Butylacetat gelöst. Beim Abkühlen auf Raumtemperatur und später auf 5°C fielen 155 g Produkt (60 % der Theorie bezogen auf eingesetztes 1,3-Dioxan-5-hydroxymethyl-5-ethyl-2-on mit einem Schmelzpunkt von 95 - 102°C) aus.

Das $^1H$-NMR-Spektrum bestätigte die Struktur und Zusammensetzung des erhaltenen Produkts als einer Verbindung der Formel (III) mit $R_1 = C_2H_5$, $R_2 = -CH_2$ 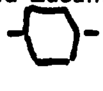 $CH_2$- und n = 2.

**Beispiel 3**

Es wurde verfahren wie in Beispiel 2, jedoch wurde das Cyclohexan-1,4-dimethanol durch die entsprechende Menge 2,2-Dimethylpropandiol-1,3 (= Neopentylglykol) ersetzt. Es wurde ein Produkt mit einem Schmelzpunkt von 74°C erhalten. Das $^1H$-NMR-Spektrum bestätigte die Struktur des erhaltenen Produkts als einer

Verbindung der Formel (III) mit $R_1 = C_2H_5$,

$$\begin{array}{c} CH_3 \\ | \\ R_2 = -CH_2-C-CH_2- \text{ und } n = 2. \\ | \\ CH_3 \end{array}$$

**Beispiel 4**

Es wurde verfahren wie in Beispiel 2, jedoch wurde das Cyclohexan-1,4-dimethanol durch die entsprechende Menge Perhydrobisphenol A ersetzt. Es wurde ein Produkt mit einem Schmelzpunkt von 190 bis 200°C erhalten. Das $^1$H-NMR-Spektrum bestätigte die Struktur des erhaltenen Produkts als einer Verbindung der Formel (III) mit $R_1 = C_2H_5$,

$R_2$ = 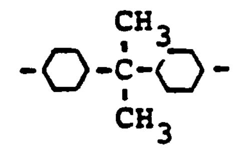 und n = 2.

**Beispiel 5**

Es wurde verfahren wie in Beispiel 2, jedoch wurde das Cyclohexan-1,4-dimethanol durch die entsprechende Menge Bisphenol A ersetzt. Es wurde ein Produkt mit einem Schmelzpunkt von 132 - 135°C erhalten. Das $^1$H-NMR-Spektrum bestätigte die Struktur des erhaltenen Produkts als einer Verbindung der Formel (III) mit $R_1$ = $C_2H_5$,

$R_2$ = 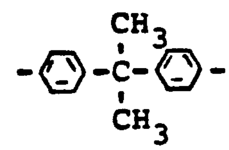 und n = 2.

**Beispiel 6**

153 g (1,18 Mol) 5,5-Dimethyl-1,3-dioxan-2-on (Formel

(IV), $R_3$ = 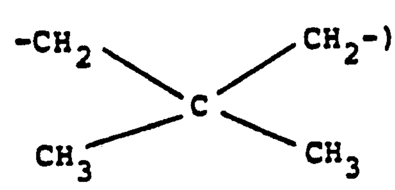 und 17 g (0,0033 Mol)

5,5'-(Cyclohexyl-1,4-bis-(methylenoxy-carboxy(oxymethylen)) bis(5-ethyl-1,3-dioxan-2-on) (Formel (III), $R_1$ = $C_2H_5$, $R_2$ = $-CH_2$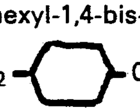$CH_2$ - und n = 2) wurden bei 120°C aufgeschmolzen. Nach Zugabe von 17 mg Thalliumacetat wurde auf 125°C erhitzt. Nach 20 Minuten trat eine Polymerisation zu einem unschmelzbaren, in Lösungsmitteln unlöslichen, transparenten Polymerisat von großer Zähe, Härte und Elastizität ein. Das Polymerisat wurde weitere 30 Minuten bei 125°C getempert.

**Beispiel 7**

Die Eigenschaften des Produktes aus Beispiel 6 und im Vergleich dazu eines Produktes, das nach dem Stand der Technik (DB-OS 31 03 139) aus 90 Gew.-% 5,5-Dimethyl-1,3-dioxan-2-on und 10 Gew.-% des Carbonates mit der Formel

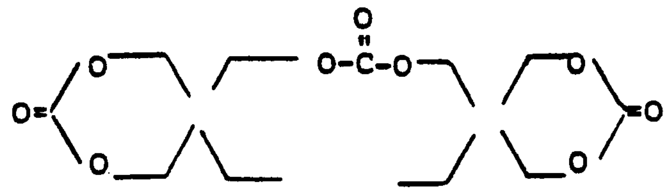

hergestellt wurde, wurden bestimmt. Die Ergebnisse sind aus Tabelle 1 ersichtlich.

Tabelle 1

| | | Stand der Technik | Produkt aus Beispiel 6 |
|---|---|---|---|
| Zugfestigkeit | R (Mpa) | 26 | 30 |
| Reißdehnung | R (%) | 12,7 | 13,6 |
| Streckspannung | S (MPa) | 22,2 | 30 |
| Streckdehnung | S (%) | 2,5 | 3,01 |
| 3,5 %-Biegespannung | b3,5 (Mpa) | 42,8 | 59 |
| Schlagzähigkeit | $a_n$ (kJ/m²) | 24,3 | 46 |
| E-Modul (Zug) | (Mpa) | 1630 | 1850 |

**Patentansprüche**

1. Mehrcyclische Kohlensäureester der Formel

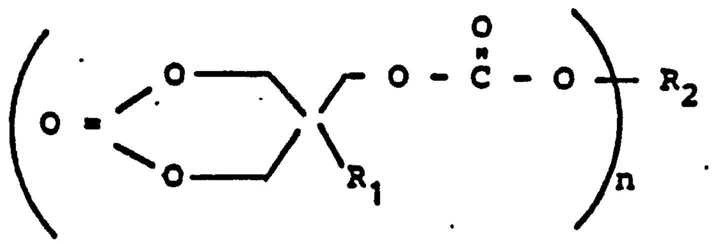

in der

$R_1$ für einen Alkylrest mit 1 bis 4 C-Atomen steht,

$R_2$ für einen aliphatischen Kohlenwasserstoff-Rest mit 3 bis 18 C-Atomen, für einen aromatischen Kohlenwasserstoff-Rest mit 6 bis 12 C-Atomen oder für einen araliphatischen Kohlenwasserstoff-Rest mit 7 bis 24 C-Atomen steht und

n 2, 3 oder 4 bedeutet.

2. Mehrcyclische Kohlensäureester der im Anspruch 1 angegebenen Formel, wobei

$R_1$ für Methyl oder Ethyl steht,

$R_2$ für das Kohlenwasserstoffgerüst eines Alkohols aus der Gruppe Trimethylolethan, Trimethylolpropan, Pentaerythrit, Butandiol-1,4, Buten-2-diol-1,4, Butin-2-diol-1,4, Neopentylglykol, Hexandiol-1,6, 2-Methylenpropandiol-1,3, Cyclohexandiol-1,4, Cyclohexan-1,4-dimethanol, Bisphenol A und Perhydrobisphenol A steht und

n die Wertigkeit des Alkohols bedeutet, dessen Kohlenwasserstoffgerüst $R_2$ darstellt.

3. Copolymere erhältlich durch Copolymerisation eines mehrcyclischen Kohlensäureesters der Formel

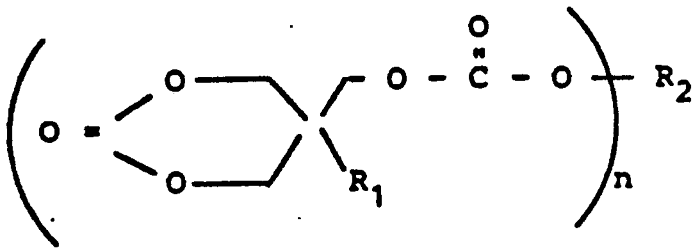

in der

$R_1$ für einen Alkylrest mit 1 bis 4 C-Atomen steht,

$R_2$ für einen aliphatischen Kohlenwasserstoff-Rest mit 3 bis 18 C-Atomen, für einen aromatischen Kohlenwasserstoff-Rest mit 6 bis 12 C-Atomen oder für einen araliphatischen Kohlenwasserstoff-Rest mit 7 bis 24 C-Atomen steht und

n 2, 3 oder 4 bedeutet,

mit monocyclischen Carbonaten der Formel

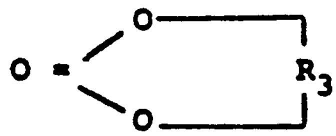

in der

$R_3$ für $(-CH_2-)_m$, wobei m für 3, 4, 5 oder 6 steht, $-CH_2-CH=CH-CH_2-$, $-CH_2-CH_2CH-(CH_3)-$, $-CH_2-CH_2-O-CH_2-CH_2-O-CH_2-CH_2-$,

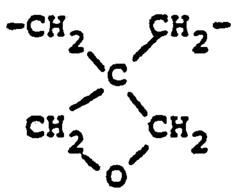 , 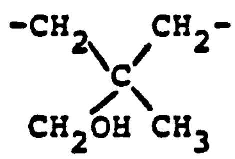 , 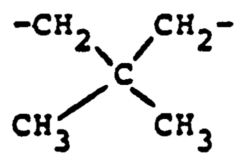 oder

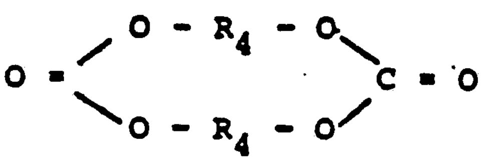

oder der Formel

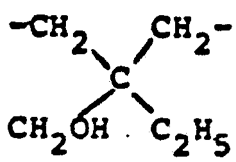 steht

in der

$R_4$ für $(-CH_2-)_p$, wobei p eine ganze Zahl zwischen 4 und 12 bedeutet, $-CH_2-CH_2-O-CH_2-CH_2-$, $-CH_2-CH_2-O-CH_2-CH_2-O-CH_2-CH_2-$ oder

$-CH_2-CH_2-N-CH_2-CH_2-$ steht,
|
$CH_3$

oder der Formel

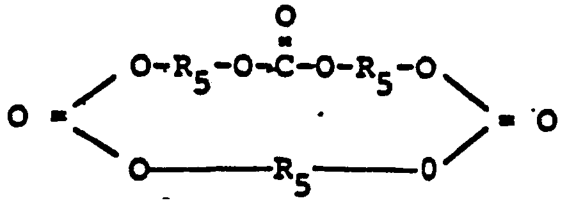

in der

$R^5$ für $-CH_2-CH_2-O-CH_2-CH_2-$ steht,

wobei man 2 bis 50 Gew.-% des mehrcyclischen Kohlensäureesters und 98 bis 50 Gew.-% des monocyclischen Carbonats einsetzt, jeweils bezogen auf die Gesamtmenge eingesetzter organischer Carbonate.

**Claims**

1. Polycyclic carbonic acid esters corresponding to the following formula

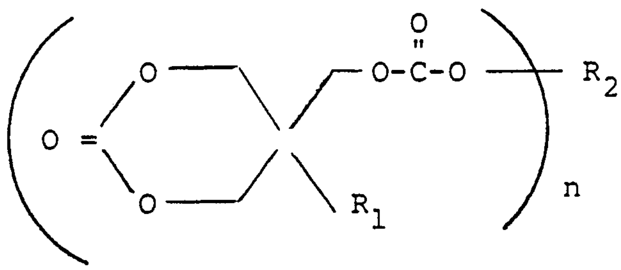

in which

$R_1$ is a $C_1$-$C_4$ alkyl radical,

$R_2$ is an aliphatic $C_3$-$C_{18}$ hydrocarbon radical, an aromatic $C_6$-$C_{12}$ hydrocarbon radical or an araliphatic $C_7$-$C_{24}$ hydrocarbon radical and

n = 2, 3 or 4.

2. Polycyclic carbonic acid esters corresponding to the formula in Claim 1, in which

$R_1$ is methyl or ethyl,

$R_2$ is the hydrocarbon residue of an alcohol from the group comprising trimethylolethane, trimethylolpropane, pentaerythritol, 1,4-butanediol, 1,4-butene-2-diol, 1,4-butyne-2-diol, neopentyl glycol, 1,6-hexanediol, 2-methyl-1,3-propanediol, 1,4-cyclohexanediol, 1,4-cyclohexane dimethanol, bisphenol A and perhydrobisphenol A and

n is the functionality of the alcohol of which $R_2$ is the hydrocarbon residue.

3. Copolymers obtainable by copolymerization of a polycyclic carbonic acid ester corresponding to the following formula

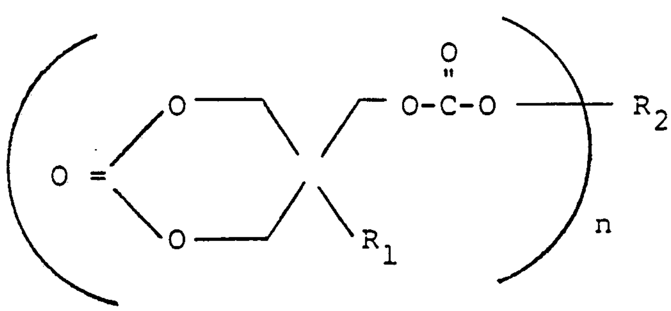

in which

$R_1$ is a $C_1$-$C_4$ alkyl radical,

$R_2$ is an aliphatic $C_3$-$C_{18}$ hydrocarbon radical, an aromatic $C_6$-$C_{12}$ hydrocarbon radical or an araliphatic $C_7$-$C_{24}$ hydrocarbon radical and

n = 2, 3 or 4,

with monocyclic carbonates corresponding to the following formula

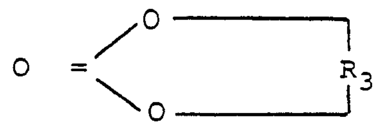

in which
$R_3$ represents $(-CH_2-)_m$, where m = 3, 4, 5 or 6, $-CH_2-CH=CH-CH_2$, $-CH_2-CH_2-CH(CH_3)-$, $-CH_2-CH_2-O-CH_2-CH_2-O-CH_2-CH_2-$

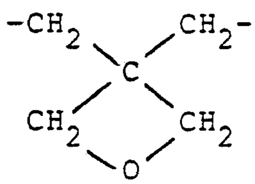 , 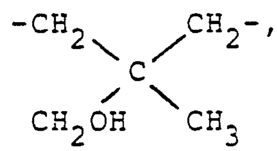 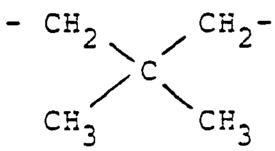 or

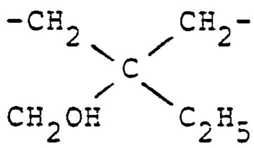

or to the following formula

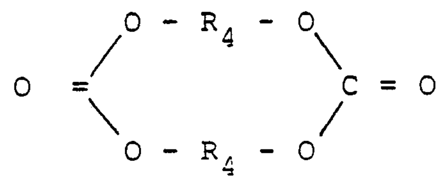

in which
$R_4$ represents $(-CH_2-)_p$, where p is an integer of from 4 to 12, $-CH_2-CH_2-O-CH_2-CH_2-$, $-CH_2-CH_2-O-CH_2-CH_2-O-CH_2-CH_2-$ or
$-CH_2-CH_2-N-CH_2-CH_2$
-         |
          $CH_3$

or to the following formula

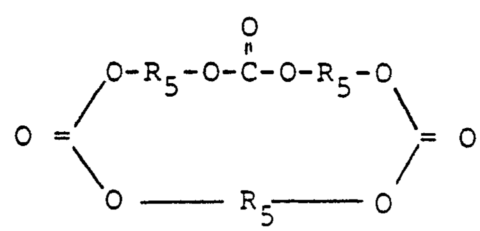

in which
$R^5$ represents $-CH_2-CH_2-O-CH_2-CH_2-$,
using from 2 to 50 % by weight of the polycyclic carbonic acid ester and from 98 to 50 % by weight of the monocyclic carbonate, based in each case of the total quantity of organic carbonates used.

## Revendications

1. Esters polycycliques d'acide carbonique de formule

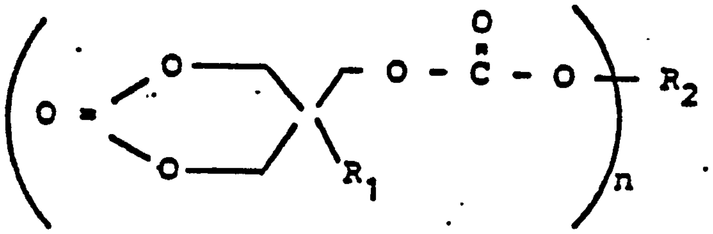

dans laquelle

$R_1$ représente un reste alkyle ayant 1 à 4 atomes de carbone,

$R_2$ est un reste d'hydrocarbure aliphatique ayant 3 à 16 atomes de carbone, un reste d'hydrocarbure aromatique ayant 6 à 12 atomes de carbone ou un reste d'hydrocarbure araliphatique ayant 7 à 24 atomes de carbone et

n a la valeur 2, 3 ou 4.

2. Esters polycycliques d'acide carbonique de formule indiquée dans la revendication 1, dans lesquels

$R_1$ est un groupe méthyle ou éthyle,

$R_2$ est le squelette hydrocarboné d'un alcool du groupe triméthyloléthane, triméthylolpropane, pentaérythritol, butanediol-1,4, butène-2-diol-1,4, butyne-2-diol-1,4, néopentylglycol, hexanediol-1,6, 2-méthylène-propanediol-1,3, cyclohexanediol-1,4, cyclohexane-1,4-diméthanol, bisphénol A et perhydrobisphénol A et

n désigne la valence de l'alcool dont $R_2$ représente le squelette hydrocarboné.

3. Copolymères pouvant être obtenus par copolymérisation d'un ester polycyclique d'acide carbonique de formule

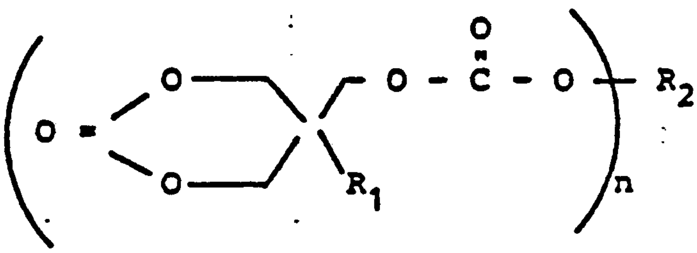

dans laquelle

$R_1$ représente un reste alkyle ayant 1 à 4 atomes de carbone,

$R_2$ est un reste d'hydrocarbure aliphatique ayant 3 à 18 atomes de carbone, un reste d'hydrocarbure aromatique ayant 6 à 12 atomes de carbone ou un reste d'hydrocarbure araliphatique ayant 7 à 24 atomes de carbone et

n a la valeur 2, 3 ou 4,

avec des carbonates monocycliques de formule

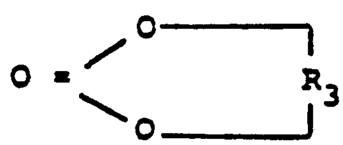

dans laquelle

$R_3$ représente un groupe $(-CH_2-)_m$, dans lequel m a la valeur 3, 4, 5 ou 6, $-CH_2-CH=CH-CH_2-$, $-CH_2-CH_2-CH(CH_3)-$, $-CH_2-CH_2-O-CH_2-CH_2-O-CH_2-CH_2-$,

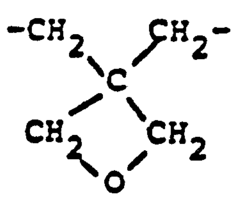 , 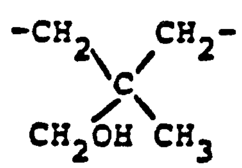 , 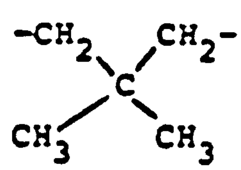 ou

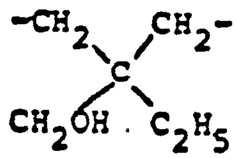

ou de formule

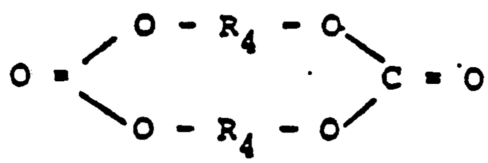

dans laquelle

$R_4$ représente $(-CH_2-)_p$, où p est un nombre entier entre 4 et 12, $-CH_2-CH_2-O-CH_2-CH_2-$, $-CH_2-CH_2-O-CH_2-CH_2-O-CH_2-CH_2-$ ou
$-CH-2-CH_2N-CH_2-CH_2-$,
|
$CH_3$

ou de formule

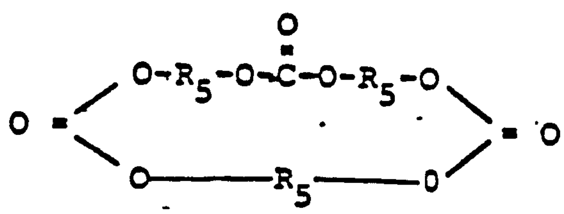

dans laquelle

$R^5$ est un groupe $-CH_2-CH_2-O-CH_2-CH_2-$,

en utilisant 2 à 50 % en poids de l'ester polycyclique de l'acide carbonique et 96 à 50 % en poids du carbonate monocyclique, dans chaque cas par rapport à la quantité totale de carbonates organiques utilisés.